# EUROPEAN PATENT APPLICATION

(11) **EP 2 452 678 A2**
(43) Date of publication of application: **16.05.2012**
(21) Application number: 11175985.8
(22) Date of filing: 29.07.2011
(51) Int. Cl.: A61K 9/50, A61K 31/4439

(54) **Pulsatile-release pharmaceutical formulation of dexlansoprazole**

(30) Priority: 30.07.2010 IN DE18082010
(71) Applicant: Ranbaxy Laboratories Limited, Delhi 110019 (IN)
(72) Inventor: Bhargava, Ankur, 122001 Gurgaon, Haryana (IN); Arora, Sachin, 122001 Gurgaon, Haryana (IN); Singla, Ajay Kumar, 160020 Chandigarh, Chandigarh (IN)
(74) Representative: Cronin, Brian Harold John

(57) **Abstract**

The present invention relates to a pulsatile-release pharmaceutical formulation of dexlansoprazole composed of a single type of enteric coated pellets and the process for the preparation thereof.

## Description

### Field of the Invention

The present invention relates to a pulsatile-release pharmaceutical formulation of dexlansoprazole composed of a single type of enteric coated pelletsand the process for the preparation thereof.

### Background of the Invention

Dexlansoprazole is the R-enantiomer of lansoprazole (a racemic mixture of the Rand S-enantiomers). Dexlansoprazole is a proton pump inhibitor that suppresses gastric acid secretion by specific inhibition of the (H+, K+)-ATPase in the gastric parietal cells. By acting specifically on the proton pump, dexlansoprazole blocks the final step of acid production. Chemically it is known as (+)-2-[(*R*)-{[3-methyl-4-(2,2,2-trifluoroethoxy)pyridin-2-yl]methyl}sulfinyl]-*1H*-benzimidazole.

Dexlansoprazole is currently marketed as Dexilant® (dexlansoprazole delayed-release capsules) which is indicated for healing of all grades of erosive esophagitis (EE), maintenance of healed EE and relief of heartburn and for the treatment of heartburn associated with non-erosive gastroesophageal reflux disease (GERD). Dexilant® is supplied as dual delayed-release formulation in which each capsule contains two types of enteric coated granules with different pH-dependent dissolution profiles. One type of granules will release the drug in the proximal region of the small intestine where the pH reaches approximately 5.5. The second type of granules will release the drug more distally in the intestine where the pH reaches approximately 6-7.5.

U.S.Patent Application No. 2006/0013868 discloses a pharmaceutical formulation of dexlansoprazole wherein the formulation comprises two kinds of granules which release at the pH of about 5.5 and a pH of about 6.0 or above, respectively.

U.S.Patent No. 7,438,929 discloses a pharmaceutical formulation comprising at least two forms of pellets, A and B, which comprise drug in the core and have different polymer coatings which determine the release of the drug at different pH values suitable for uniform release of drug in the small intestine and in the large intestine.

U.S. Patent Application No. 2009/0214599 discloses a pharmaceutical formulation wherein the formulation includes a first component to provide an initial pH dependent delayed-release of a proton pump inhibitor, and a second component to provide a pH dependent extended-release of a proton pump inhibitor. The polymers exhibiting the desired delayed-release characteristics include those dissolving at pH less than 6, and/or pH greater than 5, such as from pH 5-6. Polymers exhibiting the desired extended-release characteristics include those dissolving at a pH less than 7.5, and/or a pH greater than 6.5, for example, from pH 6.5-7.5.

U. S.Patent Application No. 2009/0098199 discloses a pharmaceutical formulation of dexlansoprazole wherein the formulation comprises at least two solid particles, each of which contain at least one proton pump inhibitor and release the drug in the proximal and distal region of the intestine, respectively.

The prior art teaches the use of two types of pellets exhibiting dual release mechanism for achieving the optimal therapeutic effect of dexlansoprazole. Therefore, a need exists to develop a formulation which will provide the desired release of dexlansoprazole from the pharmaceutical formulation but is simpler and more cost effective to manufacture. Thus, the present inventors have found that it is possible to obtain the desired dissolution profile for the final formulation without the need for the two types of pellets contemplated in the prior art. The present invention relates to a pulsatile-release pharmaceutical formulation of dexlansoprazole, wherein the formulation comprises a single type of enteric coated pellets.Use of a single type of pellet makes the formulation more economical and simpler to prepare as compared to formulations containing two types of pellets.

### Summary of the Invention

According to onegeneral aspect, the present invention provides for apulsatile-release pharmaceutical formulation of dexlansoprazole composed of single type of enteric coated pellets, wherein said enteric coated pellets include: a) a first enteric portion comprising dexlansoprazole; and b) a second enteric portion comprising dexlansoprazole, such that said first enteric portion releases dexlansoprazole in the pH range of 6-7.5 and said second enteric portion releases dexlansoprazole in the pH range of 5-6.

In another general aspect, the present invention provides for a pulsatile-release pharmaceutical formulation of dexlansoprazole composed of a single type of enteric coated pellets, wherein said enteric coated pellets include:
a) a first enteric portion which includes:
   (i) a core which includes dexlansoprazole and one or more pharmaceutically acceptable excipients;
   (ii) a barrier layer surrounding the core, said barrier layer includes a water soluble polymer and one or more pharmaceutically acceptable excipients; and
   (iii) an enteric layer surrounding the barrier layer, wherein said enteric layer includes an enteric polymer and one or morepharmaceutically acceptable excipients; such that said first enteric portion releases dexlansoprazole in the pH range of 6-7.5,and
b) a second enteric portion which includes:
   (i) a layer which includes dexlansoprazole and one or more pharmaceutically acceptable excipients;
   (ii) a barrier layer surrounding the dexlansoprazole layer, said barrier layer includes a water soluble polymer and one or more pharmaceutically acceptable excipients; and
   (iii) an enteric layer surrounding the barrier layer, wherein said enteric layer includes an enteric polymer and one or more pharmaceutically acceptable excipients; such that said second enteric portion releases dexlansoprazole in the pH range of 5-6.

In yet another general aspect, the present invention provides for a pulsatile-release pharmaceutical formulation of dexlansoprazole composed of a single type of enteric coated pellets, wherein said enteric coated pellets include:
a) a first enteric portion which includes:
   (i) an inert core;
   (ii) a drug layer surrounding the inert core which includes dexlansoprazole and one or more pharmaceutically acceptable excipients;
   (iii) a barrier layer surrounding the dexlansoprazole layer, said barrier layer includes a water soluble polymer and one or more pharmaceutically acceptable excipients; and
   (iv) an enteric layer surrounding the barrier layer, wherein said enteric layer includes an enteric polymer and one or more pharmaceutically acceptable excipients; such that said first enteric portion releases dexlansoprazole in the pH range of 6-7.5, and
b) a second enteric portion which includes:
   (i) a layer which includes dexlansoprazole and one or more pharmaceutically acceptable excipients;
   (ii) a barrier layer surrounding the dexlansoprazole layer, said barrier layer includes a water soluble polymer and one or more pharmaceutically acceptable excipients; and
   (iii) an enteric layer surrounding the barrier layer, wherein said enteric layer includes an enteric polymer and one or more pharmaceutically acceptable excipients; such that said second enteric portion releases dexlansoprazole in the pH range of 5-6.

Embodiments of the pulsatile-release pharmaceutical formulation may include one or more of the following features. For example, the first enteric portion may include 40% to 90% of dexlansoprazole and one or more pharmaceutically acceptable excipients. The second enteric portion may include 10% to 60% of dexlansoprazole and one or more pharmaceutically acceptable excipients. The first enteric portion may include an enteric polymer 10% to 50% by weight of barrier layer coated pellets.

The enteric layer of the first enteric portion may be a mixture of methacrylic acid copolymer. The enteric layer of the first enteric portion may be a mixture of methacrylic acid copolymer Type A and Type B or methacrylic acid copolymer Type A and methacrylic acid-methyl acrylate-methyl methacrylate copolymer. The enteric layer of the first enteric portion may be a mixture of methacrylic acid copolymer Type A and Type B in the ratio of 2:1 to 1:4.

The second enteric portion comprises an enteric polymer 1% to 30% by weight of barrier layer coated pellets. The enteric layer of the second enteric portion may include methacrylic acid copolymer. The enteric layer of the second enteric portion may include methacrylic acid-ethyl acrylate copolymer. The second enteric portion is coated either directly over the first enteric portion or it may be separated from the first enteric portion by a barrier layer.

In another general aspect, the present invention provides for a process to prepare the pulsatile release pharmaceutical formulation of dexlansoprazole composed of a single type of enteric coated pellets, wherein the process involves the steps of:
1) preparing the core by mixing dexlansoprazole optionally with other pharmaceutically acceptable excipients;
2) coating the core obtained in step 1 with a barrier layer which includes a water soluble polymer and one or more pharmaceutically acceptable excipients;
3) coating the resultant core of step 2 with an enteric layer to form enteric coated pellets;
4) coating the enteric coated pellets with another enteric layer by introducing an intermediate barrier layer and/or dexlansoprazole layer in between the two enteric layers; and
5) filling of the pellets obtained in step 4 into capsule after lubrication.

For example, the core may be prepared by granulation, direct blending or extrusion/spheronization.

In yet another general aspect,there is provided a process to prepare the pulsatile-release pharmaceutical formulation of dexlansoprazole composed of a single type of enteric coated pellets, wherein the process involves the steps of:
1) mixing dexlansoprazole and one or more pharmaceutically acceptable excipients to obtain a dusting powder;
2) coating an inert core with the dusting powder obtained in step 1, while spraying a binder solution to obtain dexlansoprazole core;
3) coating the core obtained in step 2 with a barrier layer which includes a water soluble polymer and one or more pharmaceutically acceptable excipients;
4) coating the resultant core of step 3 with an enteric layer to form enteric coated pellets;
5) coating the enteric coated pellets with another enteric layer by introducing an intermediate barrier layer and/or dexlansoprazole layer in between the two enteric layers;and
6) filling of the pellets obtained in step 5 into capsule after lubrication.

Embodiments of this aspect may include one or more of the following features, for example, the pulsatile-release pharmaceutical formulation may include one or more pharmaceutically acceptable excipients selected from one or more of a binder, a diluent, a surfactant, a lubricant/glidant/anti-adherent, a disintegrant, a plasticizer, a stabilizer, an opacifier/colorant/pigment, a solvent, and/or mixture thereof.

The pulsatile-release pharmaceutical formulation of the present invention is useful for therapeutics and prophylaxis of ulcer, Zollinger-Ellison's syndrome, gastritis, reflux esophagitis, symptomatic gastroesophageal reflux disease (symptomatic GERD) with no esophagitis, NUD (non-ulcer dyspepsia), gastric cancer, heartburn associated with non-erosive gastroesophageal reflux disease (GERD), maintenance and healing of erosive esophagitis, relief of heartburn, and eradication of *Helicobacter pylori.*

The pulsatile-release pharmaceutical formulation of the present invention may be used with other active ingredients selected from the group consisting of anti*-Helicobacter pylori*agents, an imidazole compound, a bismuth salt, a quinoline compound, and combinations thereof.

The pulsatile-release pharmaceutical formulation of the present invention may contain other active ingredients selected from the group consisting of anti-*Helicobacter pylori*agents, an imidazole compound, a bismuth salt, a quinoline compound, and combinations thereof.

### Detailed Description of the Invention

The pulsatile-release pharmaceutical formulation of dexlansoprazole of the present invention is composed of a single type of enteric coated pellets, wherein said enteric coated pellets include:
a) a first enteric portion which includes dexlansoprazole; and
b) a second enteric portion which includes dexlansoprazole, such that said first enteric portion releases dexlansoprazole in the pH range of 6-7.5 and said second enteric portion releases dexlansoprazole in the pH range of 5-6.

As used herein, the term "dexlansoprazole" includes the compound dexlansoprazole or its pharmaceutically acceptable salts, polymorphs, mixtures, solvates and hydrates thereof. Dexlansoprazole and its salts can be used either in crystalline form, amorphous form, or mixtures thereof.

The term "pulsatile-release" refers to the release profile in which at least two discrete quantities of dexlansoprazole are released at spaced apart time intervals depending upon the pH of the enteric polymer;*e.g*. in the present invention the first enteric layer releases dexlansoprazole in a pH range of 6-7.5 and the second enteric layer releases dexlansoprazole in the pH range of 5-6 in order to provide a pulsatile-release of dexlansoprazole which results in two distinct plasma concentration peaks.

The first enteric portion includes a dexlansoprazole core, a barrier layer surrounding the core and an enteric layer surrounding the barrier layer. The second enteric portion includes a layer that contains dexlansoprazole, a barrier layer surrounding dexlansoprazole layer and an enteric layer surrounding the barrier layer. The second enteric portion may be coated either directly over the first enteric portion or it may be separated from the first enteric portion by a barrier layer.

The term "core" as used herein, refers to a central substance that contains dexlansoprazole alone or in combination with one or more pharmaceutically acceptable excipients. The core may be in the form of inert core onto which dexlansoprazole is loaded along with other pharmaceutically acceptable excipients, using any technique such as powder layering, solution spraying, or suspension spraying. The material from which the inert core is prepared may be selected from one or more of pharmaceutically inert insoluble and/or soluble materials, with or without pharmaceutically acceptable excipients. The insoluble inert core material may be, for example, one or more of microcrystalline cellulose, plastic, glass, sand, and the like. The soluble inert core material may be, for example, one or more sugars, such as, glucose, mannitol, lactose, xylitol, dextrose, sucrose, and the like. Commercially available inert cores include sugar spheres, non pareil seeds, celpheres, and the like. Alternatively, the core can be formulated by mixing dexlansoprazole optionally with other pharmaceutically acceptable excipients followed by granulation, direct blending, extrusion/spheronization, and the like. The core may be in the form of pellets, granules or beads.

The term "barrier layer", as used herein, refers to the layer that separates dexlansoprazole containing core and the enteric layer. The purpose of the barrier layer is to prevent direct contact of dexlansoprazole with the enteric coating. The barrier layer is made up of water-soluble polymer and one or more pharmaceutically acceptable excipients. The water-soluble polymer may be selected from one or more of hydroxypropyl cellulose, hydroxypropyl methyl cellulose,methyl cellulose, hydroxyethylmethyl cellulose, polyvinyl pyrrolidone, polyvinyl alcohol, copolymer of vinylpyrrolidone and vinyl acetate, guar gum, polyethylene oxides, such as Carbopol®, Pluronic® F68,polyethylene glycol, sodium alginate, sodium carboxymethyl cellulose, and/or mixtures thereof.The barrier layer is 5% to 15% by weight of drug loaded pellets.

As used herein, the term "enteric polymer" refers to materials that are substantially insoluble at the acidic pH conditions of the stomach but are predominantly soluble in intestinal fluids at various specific pHs. The enteric polymers may be selected from the group of methyl methacrylate-methacrylic acid copolymer ((methacrylic acid copolymer Type A; which includes Eudragit® L100 &Eudragit® L 12,5- both are soluble above pH 6) or (methacrylic acid copolymer Type B; which includes Eudragit® S100 &Eudragit® S 12,5-both are soluble above pH 7)), methacrylic acid-ethyl acrylate copolymer ((Eudragit® L100-55/driedmethacrylic acid copolymer LD/methacrylic acid copolymer Type C-soluble above pH 5.5) or (Eudragit® L30D-55/methacrylic acid copolymer LD-soluble above pH 5.5)), methacrylic acid-methyl acrylate-methyl methacrylate copolymer (Eudragit® FS30D-soluble above pH 7), hydroxypropylmethyl cellulose phthalate (HP-55 & HP-55S-both are soluble above pH 5.5, HP-50-soluble above pH 5.0), cellulose acetate phthalate, cellulose acetate trimellitate, carboxymethyl ethyl cellulose, hydroxypropyl cellulose acetate succinate (HPMCAS- LF grade, MF grade and HF grade-soluble above pH 5.5, 6.0 and 6.8 respectively), polyvinyl acetate, shellac, and/or mixtures thereof.

The enteric polymers as mentioned above may be used alone or at least two or more kinds of the polymers may be used in combination in a particular ratio depending upon their dissolution pHs in order to have a desirable release profile, e.g., the enteric polymer may be a mixture of methacrylic acid copolymer Type A and Type B in the ratio of 2:1 to 1:4 or a mixture of methacrylic acid copolymer Type A and methacrylic acid-methyl acrylate-methyl methacrylate copolymer in the ratio of 1:1. The enteric polymer is present at 10% to 50% by weight of barrier layer coated pellets in the first enteric portion and 1% to 30% by weight of barrier layer coated pelletsin the second enteric portion.

The term "pharmaceutically acceptable excipients",as used herein, includes any physiologically inert, pharmacologically inactive material known to one skilled in the art, which is compatible with the physical and chemical characteristics of the active ingredient in use. The pharmaceutically acceptable excipients may be selected from one or more of a binder, a diluent, a surfactant, a lubricant/glidant/anti-adherent, a disintegrant, a plasticizer, a stabilizer, an opacifier/colorant/pigment, a solvent, and/or mixtures thereof.

Suitable binders may be selected from one or more of starches, such as corn starch, pregelatinized starch, maize starch; cellulose derivatives, such as hydroxypropylmethyl cellulose, hydroxypropyl cellulose, hydroxyethyl cellulose, methyl cellulose; gums, such as xanthan gum, gum acacia, gum arabic, tragacanth; water-soluble vinylpyrrolidone polymers, such as polyvinylpyrrolidone, copolymer of vinylpyrrolidone vinyl acetate; sugars, such as sorbitol, mannitol; sodium alginate, propylene glycol, methacrylates, and/or mixtures thereof.

Suitable diluents may be selected from one or more of sugars, such as, dextrose, glucose, sucrose, lactose; sugar alcohols, such as sorbitol, xylitol, mannitol; cellulose derivatives, such as powdered cellulose, microcrystalline cellulose, silcified microcrystalline cellulose; starches, such as corn starch, pregelatinized starch, maize starch; magnesium salts,such as oxides, carbonates, phosphates; calcium carbonate, calcium phosphate-dibasic, calcium phosphate-tribasic, calcium sulfate, and/or mixtures thereof.

Suitable surfactants may be selected from one or more of sodium lauryl sulphate, polysorbates, higher fatty acid ethers, esters, salts, and/or mixtures thereof.

The lubricants/glidants/anti-adherents may be selected from one or more of talc, aerosil, magnesium stearate, stearic acid, glyceryl monostearate, glyceryl behenate, sodium stearyl fumarate, sodium starch fumarate, and/or mixtures thereof.

Suitable disintegrants may be selected from one or more of alginic acid or alginates, microcrystalline cellulose, hydroxypropyl cellulose, carmellose, croscarmellose sodium, crospovidone, sodium starch glycolate, starch, pregelatinized starch, carboxymethyl starch, polyacrylates, and/or mixtures thereof.

Plasticizers include, for example, triethyl citrate, polyethylene glycol, triacetin, tributylsebecate, diethyl phthalate, dimethyl phthalate, propylene glycol, and/or mixtures thereof.

Suitable stabilizers may be selected from one or more of basic inorganic salt of magnesium including heavy magnesium carbonate, magnesium carbonate, magnesium oxide, magnesium hydroxide, magnesium metasilicate aluminate, magnesium silicate aluminate, magnesium silicate, magnesium aluminate, synthetic hydrotalcite and aluminum magnesium hydroxide; basic inorganic salts of calcium including precipitated calcium carbonate and calcium hydroxide; basic inorganic salts of sodium including sodium carbonate and sodium hydrogen carbonate; potassium basic inorganic salts, such as, potassium carbonate; aluminum basic inorganic salts, such as, aluminum silicate,and/or mixtures thereof.

Suitable opacifiers/pigments/colorants can be selected from one or more of titanium dioxide, iron oxide, zinc oxide, and/or mixtures thereof.

Various solvents can be used in the processes of preparation of pharmaceutical formulations of the present inventionincluding water, methanol, ethanol, acidified ethanol, acetone, diacetone, polyols, polyethers, oils, esters, alkyl ketones, methylene chloride, isopropyl alcohol, butyl alcohol, methyl acetate, ethyl acetate, isopropyl acetate, castor oil, ethylene glycol monoethyl ether, diethylene glycol monobutyl ether, diethylene glycol monoethyl ether, dimethylsulphoxide, dimethylformamide, tetrahydrofuran, and/or mixtures thereof.

The enteric coated pellets, granules or beads are mixed with one or more pharmaceutically acceptable excipients and are either filled into a capsuleor compressed into a tablet for ease of administration.

Optionally, the pharmaceutical formulations can be further coated with a thin film. Frequently, the film will be colored to facilitate product identification and for aesthetic purposes. Many suitable film coating products are commercially available including the Opadry® and Opaglos®products.

The barrier layer, enteric layer or optionally the over coating layer can be applied on to the dexlansoprazole containing core by coating or by layering procedures in suitable equipments,such as, a coating pan, a wurster coater, a centrifugal coater, a coating granulator or in a fluidized bed apparatus.

The pharmaceutical formulation of dexlansoprazole may be bioequivalent to the delayed-release pharmaceutical formulation of dexlansoprazole, marketed in the United States under the brand name Dexilant^{®} by Takeda Pharmaceuticals.

The term "bioequivalent", as used herein, denotes a scientific basis on which generic and brand name drugs are compared with one another. In the present context, two formulations are regarded as bioequivalent if the value of the parameter at 90% confidence interval is within 80% to 125% of that of a similar commercially available product used as reference (as per FDA, EMEA, NIHS& Health Canada).

The process to prepare the pulsatile-release pharmaceutical formulation of dexlansoprazole composed of a single type of enteric coated pellets involves the steps of:
1) preparing the core by mixing dexlansoprazole optionally with other pharmaceutically acceptable excipients;
2) coating the core obtained in step 1 with a barrier layer which includes a water soluble polymer and one or more pharmaceutically acceptable excipients;
3) coating the resultant core of step 2 with an enteric layer to form enteric coated pellets;
4) coating the enteric coated pellets with another enteric layer by introducing an intermediate barrier layer and/or dexlansoprazole layer in between the two enteric layers; and
5) filling of the pellets obtained in step 4 into a capsule after lubrication.

The process to prepare the pulsatile-release pharmaceutical formulation of dexlansoprazole composed of a single type of enteric coated pellets, may also involve the steps of:
1) mixing dexlansoprazole and one or more pharmaceutically acceptable excipients to obtain a dusting powder;
2) coating an inert core with the dusting powder obtained in step 1, while spraying a binder solution to obtain dexlansoprazole core;
3) coating the core obtained in step 2 with a barrier layer which includes a water soluble polymer and one or more pharmaceutically acceptable excipients;
4) coating the resultant core of step 3 with an enteric layer to form enteric coated pellets;
5) coating the enteric coated pellets with another enteric layer by introducing an intermediate barrier layer and/or dexlansoprazole layer in between the two enteric layers;and
6) filling of the pellets obtained in step 5 into a capsule after lubrication.

The pharmaceutical formulation of the present invention is useful in mammals for the treatment and prevention of ulcer (*e.g*., gastric ulcer, digestive ulcer, duodenal ulcer, marginal ulcer etc.),Zollinger-Ellison syndrome, gastritis, reflux esophagitis, symptomatic gastroesophageal reflux disease (symptomatic GERD) with no esophagitis, NUD (non-ulcer dyspepsia), gastric cancer and gastric MALT lymphoma, heartburn associated with non-erosive gastroesophageal reflux disease (GERD), maintenance and healing of erosive esophagitis, relief of heartburn, *Helicobacter pylori* eradication, suppression of upper gastrointestinal hemorrhage due to digestive ulcer, acute stress ulcer and hemorrhagic gastritis, suppression of upper gastrointestinal hemorrhage due to invasive stress (stress from major surgery necessitating intensive management after surgery, and from cerebral vascular disorder, head trauma, multiple organfailure and extensive burns necessitating intensive treatment), treatment and prevention of ulcer caused by a non-steroidal antiinflammatory agent, treatment and prevention of hyperacidity and ulcer due to postoperative stress, pre-anesthetic administration, etc.

The pharmaceutical formulation of the present invention may also be used in combination with other active ingredients, including anti*-Helicobacter pylori* agents, imidazole compounds, bismuth salts, quinolone compounds, and so forth. Of these substances, anti*-Helicobacter pylori* agents, and imidazole compounds,are preferred. "Anti*-Helicobacter pylori* agents" include antibiotic penicillins (*e.g*. amoxicillin, benzylpenicillin, piperacillin, mecillinam, etc.), antibiotic cefems (*e.g*. cefixime, cefaclor, etc.), antibiotic macrolides (*e.g.* erythromycin, clarithromycin. etc.), antibiotic tetracyclines (*e.g*. tetracycline, minocycline, streptomycin, etc.), antibiotic aminoglycosides (*e.g*. gentamicin, amikacin, etc.), and imipenem. Of these substances, antibiotic penicillins, and antibiotic macrolides are preferred. "Imidazole compounds" include metronidazole, miconazole, etc. "Bismuth salts" include bismuth acetate, bismuth citrate, etc. "Quinolone compounds" include ofloxacin, ciploxacin, etc.

The pulsatile-release pharmaceutical formulation of the present invention may contain other active ingredients selected from the group consisting of anti*-Helicobacter pylori* agents, an imidazole compound, a bismuth salt, a quinoline compound, andcombinations thereof.

Such "other active ingredients" and the pharmaceutical formulation of present invention may also be used in combination as a mixture prepared as a single pharmaceutical composition [*e.g*. tablets, powders, granules, capsules (including soft capsules), liquids, injectable preparations, suppositories, sustained-release preparations, etc.], in accordance with a commonly known method, and may also be prepared as separate preparations and administered to the same subject simultaneously or at a time interval.

The invention is further defined by reference to the following non-limiting examples illustrating in detail the preparation of the pharmaceutical formulation of dexlansoprazole disclosed in various embodiments of the specification. It will be apparent to those skilled in the art that many modifications, both to materials and methods, may be practiced without departing from the purpose and interest of this invention.

### Example 1:

| **Ingredient** | **Quantity (mg/Capsule)** | |
|---|---|---|
| **Strengths** | **60** | **30** |
| **1st Drug Layer** | | |
| Sugar Beads | 38.00 | 19.00 |
| Dexlansoprazole | 45.00 | 22.50 |
| Magnesium Carbonate | 12.00 | 6.00 |
| Sucrose | 30.00 | 15.00 |
| Low Substituted Hydroxypropyl Cellulose | 9.00 | 4.50 |
| Hydroxypropyl Cellulose | 3.00 | 1.50 |
| Solvent | q. s. | q. s. |

| **1st BarrierLayer** | | |
|---|---|---|
| Hydroxypropylmethyl Cellulose | 9.40 | 4.70 |
| Talc | 3.00 | 1.50 |
| Titanium Dioxide | 4.00 | 2.00 |
| Solvent | q. s. | q. s. |

| **1st Enteric Layer (Soluble at pH 6-7.5)** | | |
|---|---|---|
| Talc | 21.00 | 10.50 |
| Eudragit® L100 | 21.00 | 10.50 |
| Eudragit® S100 | 21.00 | 10.50 |
| Triethyl Citrate | 4.00 | 2.00 |
| Solvent | q. s. | q.s. |

| **2nd Barrier Layer** | | |
|---|---|---|
| Hydroxypropylmethyl Cellulose | 10.00 | 5.00 |
| Talc | 3.00 | 1.50 |
| Titanium Dioxide | 4.00 | 2.00 |
| Solvent | q. s. | q.s. |

| **2nd Drug Layer** | | |
|---|---|---|
| Dexlansoprazole | 15.00 | 7.50 |
| Magnesium Carbonate | 3.50 | 1.75 |
| Sucrose | 11.00 | 5.50 |
| Low Substituted Hydroxypropyl Cellulose | 2.50 | 1.25 |
| Hydroxypropyl Cellulose | 0.96 | 0.48 |
| Solvent | q. s. | q. s. |

| **3rd Barrier Layer** | | |
|---|---|---|
| Hydroxypropylmethyl Cellulose | 2.94 | 1.47 |
| Talc | 1.00 | 0.50 |
| Titanium Dioxide | 2.00 | 1.00 |
| Solvent | q. s. | q. s. |

| **2nd Enteric Layer(Soluble at pH 5-6)** | | |
|---|---|---|
| Titanium Dioxide | 1.00 | 0.50 |
| Talc | 2.00 | 1.00 |
| Eudragit®L30 D55 | 7.20 | 3.60 |
| Triethyl Citrate | 1.50 | 0.75 |
| Solvent | q. s. | q.s. |

| **Lubrication** | | |
|---|---|---|
| Aerosil | 1.00 | 0.50 |
| Talc | 1.00 | 0.50 |
| **Total Fill Weight** | **290.00** | **145.00** |

### Process:

1. Dexlansoprazole, magnesium carbonate, sucrose and low substituted hydroxypropyl cellulose were mixed well to obtain a dusting powder.
2. Sugar beads were coated with the above dusting powder while spraying ahydroxypropyl cellulose solution.
3. The coating dispersion for the first barrier layer was prepared by dissolving hydroxypropylmethyl cellulose in the solvent followed by dispersing titanium oxide and talc into the obtained dispersion.
4. The pellets obtained in step 2 were coated with the coating dispersion obtained in step 3.
5. Eudragit® L100, Eudragit® S100 and triethyl citrate were dissolved in the solvent and talc was dispersed into the resulting dispersion to obtain an enteric coating dispersion.
6. The pellets obtained in step 4 were coated with the above enteric coating dispersion prepared in step 5 to give enteric coated pellets.
7. The coating dispersion for the second barrier layer was produced by dissolving hydroxypropylmethyl cellulose in the solvent followed by dispersing titanium oxide and talc into the obtained dispersion.
8. The pellets obtained in step 6 were coated with the coating dispersion obtained in step 7.
9. Dexlansoprazole, magnesium carbonate, sucrose and low substituted hydroxypropyl cellulose were mixed well to obtain a dusting powder for second drug layer.
10. The pellets obtained in step 8 were layered with the dusting powder obtained in step 9 while spraying the hydroxypropyl cellulose solution.
11. The coating dispersion for the third barrier layer was produced by dissolving hydroxypropylmethyl cellulose in the solvent followed by dispersing titanium oxide and talc into the obtained dispersion.
12. The pellets obtained in step 10 were further coated with the coating dispersion obtained in step 11.
13. Eudragit® L30 D55 and triethyl citrate were dissolved in the solvent and talc was dispersed into the resulting dispersion to obtain an enteric coating dispersion.
14. The pellets obtained in step 12 were further coated with the enteric coating dispersion obtained in step 13.
15. To the resultant enteric coated pellets, talc and aerosil were added to give lubricated enteric coated pellets which were filled into capsule.

### Example 2:

| **Ingredient** | **Quantity (mg/Capsule)** | |
|---|---|---|
| **Strengths** | **60** | **30** |
| **1st Drug layer** | | |
| Cellulose Beads | 38.00 | 19.00 |
| Dexlansoprazole | 45.00 | 22.50 |
| Magnesium Carbonate | 12.00 | 6.00 |
| Sucrose | 30.00 | 15.00 |
| Low Substituted Hydroxypropyl Cellulose | 9.00 | 4.50 |
| Hydroxypropyl Cellulose | 3.00 | 1.50 |
| Solvent | q. s. | q. s. |

| **1st Barrier Layer** | | |
|---|---|---|
| Hydroxypropyl Methyl Cellulose | 9.40 | 4.70 |
| Talc | 3.00 | 1.50 |
| Titanium Dioxide | 4.00 | 2.00 |
| Solvent | q. s. | q. s. |

| **1st Enteric Layer (Soluble at pH 6-7.5)** | | |
|---|---|---|
| Talc | 21.00 | 10.50 |
| Eudragit® L100 | 21.00 | 10.50 |
| Eudragit® FS30D | 21.00 | 10.50 |
| Triethyl Citrate | 4.00 | 2.00 |
| Solvent | q. s. | q. s. |

| **2nd Barrier Layer** | | |
|---|---|---|
| Hydroxypropylmethyl Cellulose | 10.00 | 5.00 |
| Talc | 3.00 | 1.50 |
| Titanium Dioxide | 4.00 | 2.00 |
| Solvent | q. s. | q. s. |

| **2nd Drug layer** | | |
|---|---|---|
| Dexlansoprazole | 15.00 | 7.50 |
| Magnesium Carbonate | 3.50 | 1.75 |
| Sucrose | 11.00 | 5.50 |
| Low Substituted Hydroxypropyl Cellulose | 2.50 | 1.25 |
| Hydroxypropyl Cellulose | 0.96 | 0.48 |
| Solvent | q. s. | q. s. |

| **3rd Barrier Layer** | | |
|---|---|---|
| Hydroxypropyl Methyl Cellulose | 2.94 | 1.47 |
| Talc | 1.00 | 0.50 |
| Titanium Dioxide | 2.00 | 1.00 |
| Solvent | q. s. | q. s. |

| **2nd Enteric Laver (Soluble at pH 5-6)** | | |
|---|---|---|
| Titanium Dioxide | 1.00 | 0.50 |
| Talc | 2.00 | 1.00 |
| Eudragit® L30 D55 | 7.20 | 3.60 |
| Triethyl Citrate | 1.50 | 0.75 |
| Polysorbate | 1.00 | 0.50 |
| Solvent | q. s. | q. s. |

| **Lubrication** | | |
|---|---|---|
| Talc | 1.00 | 0.50 |
| **Total Fill Weight** | **290.00** | **145.00** |

### Process:

1. Dexlansoprazole, magnesium carbonate, sucrose and low substituted hydroxypropyl cellulose were mixed well to obtain a dusting powder.
2. Cellulose beads were coated with the above dusting powder while spraying a hydroxypropyl cellulose solution.
3. The coating dispersion for the first barrier layer was produced by dissolving hydroxypropyl methyl cellulose in the solvent followed by dispersing titanium oxide and talc into the obtained dispersion.
4. The pellets obtained in step 2 were coated with the coating dispersion obtained in step 3.
5. Eudragit® L 100, Eudragit® FS 30 D and triethyl citrate were dissolved in the solvent and talc was dispersed into the resulting dispersion to obtain an enteric coating dispersion.
6. The pellets obtained in step 4 were coated with the above enteric coating dispersion prepared in step 5 to give enteric coated pellets.
7. The coating dispersion for the second barrier layer was produced by dissolving hydroxypropylmethyl cellulose in the solvent followed by dispersing titanium oxide and talc into the obtained dispersion.
8. The pellets obtained in step 6 were coated with the coating dispersion obtained in step 7.
9. Dexlansoprazole, magnesium carbonate, sucrose and low substituted hydroxypropyl cellulose were mixed well to obtain a dusting powder for second drug layer.
10. The pellets obtained in step 8 were layered with the dusting powder obtained in step 9 while spraying the hydroxypropyl cellulose solution.
11. The coating dispersion for the third barrier layer was produced by dissolving hydroxypropyl methyl cellulose in the solvent followed by dispersing titanium oxide and talc into the obtained dispersion.
12. The pellets obtained in step 10 were further coated with the coating dispersion obtained in step 11.
13. Eudragit® L30 D55, polysorbate and triethyl citrate were dissolved in the solvent and talc was dispersed into the resulting dispersion to obtain an enteric coating dispersion.
14. The pellets obtained in step 12 were further coated with the enteric coating dispersionobtained in step 13.
15. To the resultant enteric coated pellets, talc was added to give lubricated enteric coated pellets which were filled into capsule.

### Example 3:

| **Ingredient** | **Quantity (mg/Capsule)** | |
|---|---|---|
| **Strengths** | **60** | **30** |
| **1st Drug layer** | | |
| Sugar Pellets | 40.00 | 20.00 |
| Dexlansoprazole | 45.00 | 22.50 |
| Magnesium Carbonate Heavy | 12.00 | 6.00 |
| Confectioner's Sugar | 37.50 | 18.75 |
| Low Substituted Hydroxypropyl Cellulose | 22.50 | 11.25 |
| Hydroxypropyl Cellulose | 1.50 | 0.75 |
| Water Alcohol Mixture | q. s. | q. s. |
| **Total** | **158.50** | **79.25** |

| **1st Barrier Layer** | | |
|---|---|---|
| 1^{st} Drug Layered Pellet | 158.50 | 79.25 |
| Hydroxypropylmethyl Cellulose | 8.72 | 4.36 |
| Talc | 4.75 | 2.37 |
| Titanium Dioxide | 2.38 | 1.19 |
| Water Alcohol Mixture | q. s. | q. s. |
| **Total** | **174.35** | **87.17** |

| **1st Enteric Laver (Soluble at pH 6-7.5)** | | |
|---|---|---|
| 1^{st}Barrier Layer Coated Pellet | 174.35 | 87.17 |
| Talc | 15.35 | 7.67 |
| Eudragit® L100 | 10.96 | 5.48 |
| Eudragit® S100 | 32.89 | 16.44 |
| Triethyl Citrate | 6.58 | 3.29 |
| Ethanol | q. s. | q. s. |
| Water | q. s. | q. s. |
| **Total** | **240.13** | **120.06** |

| **2nd Barrier Layer** | | |
|---|---|---|
| 1^{st}Enteric Layer Coated Pellet | 240.13 | 120.06 |
| Hydroxypropylmethyl Cellulose | 13.21 | 6.60 |
| Talc | 7.20 | 3.60 |
| Titanium Dioxide | 3.60 | 1.80 |
| Water Alcohol Mixture | q. s. | q. s. |
| **Total** | **264.14** | **132.07** |

| **2nd Drug layer** | | |
|---|---|---|
| 2^{nd}Barrier Layer Coated Pellet | 264.14 | 132.07 |
| Dexlansoprazole | 15.00 | 7.50 |
| Magnesium Carbonate Heavy | 4.00 | 2.00 |
| Confectioner's Sugar | 12.50 | 6.25 |
| Low Substituted Hydroxypropyl Cellulose | 5.00 | 2.50 |
| Hydroxypropyl Cellulose | 0.50 | 0.25 |
| Water Alcohol Mixture | q. s. | q. s. |
| **Total** | **301.14** | **150.57** |

| **3rd Barrier Layer** | | |
|---|---|---|
| 2^{nd} Drug Layered Pellet | 301.14 | 150.57 |
| Hydroxypropylmethyl Cellulose | 16.56 | 8.28 |
| Talc | 9.03 | 4.51 |
| Titanium Dioxide | 4.52 | 2.26 |
| Water Alcohol Mixture | q. s. | q. s. |
| **Total** | **331.25** | **165.62** |

| **2nd Enteric Layer (Soluble at pH 5-6)** | | |
|---|---|---|
| 3^{rd} Barrier Layer Coated Pellet | 331.25 | 165.62 |
| Eudragit® L30 D55 | 28.16 | 14.08 |
| Polysorbate 80 | 0.28 | 0.14 |
| Triethyl Citrate | 4.22 | 2.11 |
| Talc | 12.50 | 6.25 |
| Water | q. s. | q. s. |
| **Total** | **376.41** | **188.20** |

| **Lubrication** | | |
|---|---|---|
| 2^{nd} Enteric Layer Coated Pellet | 376.41 | 188.20 |
| Aerosil | 1.90 | 0.95 |
| Talc | 1.90 | 0.95 |
| **Total Fill Weight** | **380.21** | **190.10** |

| **Capsule filling** | | |
|---|---|---|
| Lubricated Pellet | 380.21 | 190.10 |
| Hydroxypropyl Methylcellulose Capsule | 1 No. | 1 No. |

### Process:

1. Dexlansoprazole, magnesium carbonate heavy, sugar and low substituted hydroxypropyl cellulose were sifted through a suitable screen and mixed well to obtain a dusting powder.
2. Hydroxypropyl cellulose was dissolved in a water alcohol mixture to form binder solution.
3. Sugar pellets were layered with the dusting powder obtained in step 1 while spraying thebinder solution obtained in step 2.
4. The coating dispersion for the first barrier layer was prepared by dissolving hydroxypropylmethyl cellulose in the water alcohol mixture followed by dispersing titanium oxide and talc into the obtained dispersion.
5. The pellets obtained in step 3 were coated with the coating dispersion obtained in step 4.
6. Eudragit® L100, Eudragit® S100 and triethyl citrate were dissolved in the water alcohol mixture and talc was dispersed into the resulting dispersion to obtain an enteric coating dispersion.
7. The pellets obtained in step 5 were coated with the above enteric coating dispersion prepared in step 6 to give enteric coated pellets.
8. The coating dispersion for the second barrier layer was produced by dissolving hydroxypropylmethyl cellulose in the water alcohol mixture followed by dispersing titanium oxide and talc into the obtained dispersion.
9. The pellets obtained in step 7 were coated with the coating dispersion obtained in step 8.
10. Dexlansoprazole, magnesium carbonate heavy, sugar and low substituted hydroxypropyl cellulose were sifted and mixed well to obtain a dusting powder for second drug layer.
11. The pellets obtained in step 9 were layered with the dusting powder obtained in step 10 while spraying the hydroxypropyl cellulose solution.
12. The coating dispersion for the third barrier layer was produced by dissolving hydroxypropylmethyl cellulose in the water alcohol mixture followed by dispersing titanium oxide and talc into the obtained dispersion.
13. The pellets obtained in step 10 were further coated with the coating dispersion obtained in step 12.
14. To prepare the enteric coating dispersion, triethyl citrate, polysorbate and talc were allowed to disperse in a portion of water and this was added to Eudragit® L30 D55 while stirring and then rest of water was added.
15. The pellets obtained in step 13 were further coated with the enteric coating dispersionobtained in step 14.
16. To the resultant enteric coated pellets, talc and aerosil were added to give lubricated enteric coated pellets which were filled into HPMC capsule (1 No).

### Example 4:

| **Ingredient** | **Quantity (mg/Capsule)** | |
|---|---|---|
| **Strengths** | **60** | **30** |
| **1st Drug layer** | | |
| Sugar Pellets | 40.00 | 20.00 |
| Dexlansoprazole | 45.00 | 22.50 |
| Magnesium Carbonate Heavy | 9.00 | 4.50 |
| Sugar | 37.50 | 18.75 |
| Low Substituted Hydroxypropyl Cellulose | 22.50 | 11.25 |
| Hydroxypropyl Cellulose | 1.00 | 0.50 |
| Water | q. s. | q. s. |
| **Total** | **155.00** | **77.50** |

| **1st Barrier Layer** | | |
|---|---|---|
| 1^{st} Drug Layered Pellet | 155.00 | 77.50 |
| Hydroxypropylmethyl Cellulose | 10.23 | 5.11 |
| Talc | 5.58 | 2.79 |
| Titanium dioxide | 2.79 | 1.40 |
| Water | q. s. | q. s. |
| **Total** | **173.60** | **86.80** |

| **1st Enteric Layer (Soluble at pH 6-7.5)** | | |
|---|---|---|
| 1^{st} Barrier Layer Coated Pellet | 173.60 | 86.80 |
| Talc | 19.44 | 9.72 |
| Eudragit® L100 | 13.89 | 6.94 |
| Eudragit® S100 | 41.66 | 20.83 |
| Triethyl Citrate | 7.78 | 3.89 |
| Ethanol | q. s. | q. s. |
| Water | q. s. | q. s. |
| **Total** | **256.37** | **128.18** |

| **2nd Barrier Layer** | | |
|---|---|---|
| 1^{st}Enteric Layer Coated Pellet | 256.37 | 128.18 |
| Hydroxypropylmethyl Cellulose | 16.78 | 8.39 |
| Talc | 9.15 | 4.57 |
| Titanium Dioxide | 4.58 | 2.29 |
| Water | q. s. | q. s. |
| **Total** | **286.88** | **143.44** |

| **2nd Drug layer** | | |
|---|---|---|
| 2^{nd}Barrier Layer Coated Pellet | 284.81 | 142.40 |
| Dexlansoprazole | 15.00 | 7.50 |
| Magnesium Carbonate | 4.00 | 2.00 |
| Sugar | 12.50 | 6.25 |
| Low Substituted Hydroxypropyl Cellulose | 10.00 | 5.00 |
| Hydroxypropyl Cellulose | 0.30 | 0.15 |
| Water | q. s. | q. s. |
| **Total** | **326.61** | **163.30** |

| **3rd Barrier Layer** | | |
|---|---|---|
| 2^{nd} Drug Layered Pellet | **326.61** | **163.30** |
| Hydroxypropylmethyl Cellulose | 20.67 | 10.33 |
| Talc | 11.28 | 5.64 |
| Titanium Dioxide | 5.64 | 2.82 |
| Water | q. s. | q. s. |
| **Total** | **364.20** | **182.10** |

| **2nd Enteric Layer (Soluble at pH 5-6)** | | |
|---|---|---|
| 3^{rd}Barrier Layer Coated Pellet | **364.20** | **182.10** |
| Eudragit® L 30 D55 | 26.77 | 13.38 |
| Polysorbate 80 | 0.27 | 0.14 |
| Triethyl Citrate | 4.02 | 2.01 |
| Talc | 16.06 | 8.03 |
| Water | q. s. | q. s. |
| **Total** | **411.33** | **205.61** |

| **Lubrication** | | |
|---|---|---|
| 2^{nd}Enteric Layer Coated Pellet | 411.33 | 205.61 |
| Aerosil | 1.02 | 0.51 |
| Talc | 1.02 | 0.51 |
| **Total Fill Weight** | **413.37** | **206.18** |

| **Capsule Filling** | | |
|---|---|---|
| Lubricated Pellet | 413.37 | 206.18 |
| Hydroxypropyl Methylcellulose Capsule | 1 No. | 1 No. |

### Process:

1. Dexlansoprazole, magnesium carbonate, sugar and low substituted hydroxypropyl cellulose were sifted through a suitable screen and mixed well to obtain a dusting powder.
2. Hydroxypropyl cellulose was dissolved in a water to form binder solution.
3. Sugar pellets were layered with the dusting powder obtained in step 1 while spraying the binder solution obtained in step 2.
4. The coating dispersion for the first barrier layer was prepared by dissolving hydroxypropylmethyl cellulose in the water followed by dispersing titanium oxide and talc into the obtained dispersion.
5. The pellets obtained in step 3 were coated with the coating dispersion obtained in step 4.
6. Eudragit® L100, Eudragit® S100 and triethyl citrate were dissolved in the water and ethanol and talc was dispersed into the resulting dispersion to obtain an enteric coating dispersion.
7. The pellets obtained in step 5 were coated with the above enteric coating dispersion prepared in step 6 to give enteric coated pellets.
8. The coating dispersion for the second barrier layer was produced by dissolving hydroxypropylmethyl cellulose in the water followed by dispersing titanium oxide and talc into the obtained dispersion.
9. The pellets obtained in step 7 were coated with the coating dispersion obtained in step 8.
10. Dexlansoprazole, magnesium carbonate, sugar and low substituted hydroxypropyl cellulose were sifted and mixed well to obtain a dusting powder for second drug layer.
11. The pellets obtained in step 9 were layered with the dusting powder obtained in step 10 while spraying the hydroxypropyl cellulose solution.
12. The coating dispersion for the third barrier layer was produced by dissolving hydroxypropylmethyl cellulose in the water followed by dispersing titanium oxide and talc into the obtained dispersion.
13. The pellets obtained in step 10 were further coated with the coating dispersion obtained in step 12.
14. To prepare the enteric coating dispersion,triethyl citrate, polysorbate 80 and talc were allowed to disperse in a portion of water and this was added to Eudragit® L30 D55 while stirring and then rest of water was added.
15. The pellets obtained in step 13 were further coated with the enteric coating dispersionobtained in step 14.
16. To the resultant enteric coated pellets, talc and aerosil were added to give lubricated enteric coated pellets which were filled into HPMC capsule (1 No).

### In-Vitro Dissolution Profile:

The formulation of dexlansoprazole DR Capsules obtained in Example 4 was subjected to dissolution studies first in 500 ml of 0.1N HCl at 37°C using USP Apparatus I (Basket method) at 100 rpm for 2 hours and then in 900 ml of phosphate buffer (pH 7.0) with 5mM SLS at 37°C using USP apparatus I (Basket method) at 100 rpm. Table 1 provides the dissolution profile.

**Table 1:**

| **Time Point (Min)** | **% Drug Dissolved** |
|---|---|
| **Dissolution in 0.1N HCl** | |
| 120 | <1 |

| **Dissolution in Phosphate Buffer (pH 7.0)+SLS** | |
|---|---|
| 130 | 20 |
| 140 | 23 |
| 160 | 63 |
| 170 | 93 |
| 180 | 97 |
| 195 | 99 |
| 225 | 100 |
| 240 | 100 |

### Example 5:

| **Ingredient** | **Quantity (mg/Capsul**e) | |
|---|---|---|
| **Strengths** | **60** | **30** |
| **1st Drug Layer** | | |
| Sugar Pellets | 40.00 | 20.00 |
| Dexlansoprazole | 45.00 | 22.50 |
| Magnesium Carbonate Heavy | 9.00 | 4.50 |
| Sugar | 37.50 | 18.75 |
| Low Substituted Hydroxypropyl Cellulose | 22.50 | 11.25 |
| Hydroxypropyl Cellulose | 1.00 | 0.50 |
| Water | q. s. | q. s. |
| **Total** | **155.00** | **77.50** |

| **1st Barrier Layer** | | |
|---|---|---|
| 1^{st}Drug Layered Pellet | 155.00 | 77.50 |
| Hydroxypropylmethyl Cellulose | 10.23 | 5.11 |
| Talc | 5.58 | 2.79 |
| Titanium Dioxide | 2.79 | 1.40 |
| Water | q. s. | q. s. |
| **Total** | **173.60** | **86.80** |

| **1st Enteric Layer (Soluble at pH 6-7.5)** | | |
|---|---|---|
| 1^{st}Barrier Layer Coated Pellet | 173.60 | 86.80 |
| Talc | 19.44 | 9.72 |
| Eudragit®L100 | 13.89 | 6.94 |
| Eudragit®S100 | 41.66 | 20.83 |
| Triethyl Citrate | 7.22 | 3.61 |
| Ethanol | q. s. | q. s. |
| Water | q. s. | q. s. |
| **Total** | **255.82** | **127.91** |

| **2nd Barrier Layer** | | |
|---|---|---|
| 1^{st}Enteric Layer Coated Pellet | **257.31** | **128.65** |
| Hydroxypropylmethyl Cellulose | 16.98 | 8.49 |
| Talc | 9.26 | 4.63 |
| Titanium Dioxide | 4.63 | 2.21 |
| Water | q. s. | q. s. |
| **Total** | **288.19** | **144.09** |

| **2nd Drug layer** | | |
|---|---|---|
| 2^{nd}Barrier Layer Coated Pellet | 290.17 | 144.09 |
| Dexlansoprazole | 15.00 | 7.50 |
| Magnesium Carbonate | 4.00 | 2.00 |
| Sugar | 12.50 | 6.25 |
| Low Substituted Hydroxypropyl Cellulose | 10.00 | 5.00 |
| Hydroxypropyl Cellulose | 0.30 | 0.15 |
| Water | q. s. | q. s. |
| **Total** | **331.97** | **165.93** |

| **3rd Barrier Layer** | | |
|---|---|---|
| 2^{nd}Drug Layered Pellet | **331.97** | **165.93** |
| Hydroxypropylmethyl Cellulose | 21.91 | 10.85 |
| Talc | 11.95 | 5.97 |
| Titanium Dioxide | 5.98 | 2.99 |
| Water | q. s. | q. s. |
| **Total** | **371.81** | **185.90** |

| **2nd Enteric Layer (Soluble at pH 5-6)** | | |
|---|---|---|
| 3^{rd} Barrier layer coated pellet | **374.80** | **187.40** |
| Eudragit® L30 D55 | 29.98 | 14.99 |
| Polysorbate 80 | 0.30 | 0.15 |
| Triethyl citrate | 4.50 | 2.25 |
| Talc | 17.99 | 8.99 |
| Water | q. s. | q. s. |
| **Total** | **427.57** | **213.78** |

| **Lubrication** | | |
|---|---|---|
| 2^{nd}Enteric Layer Coated Pellet | 427.57 | 213.78 |
| Aerosil | 1.00 | 0.50 |
| Talc | 1.00 | 0.50 |
| **Total Fill Weight** | **429.57** | **214.78** |

| **Capsule Filling** | | |
|---|---|---|
| Lubricated Pellet | **429.57** | **214.78** |
| Hydroxypropyl Methylcellulose Capsule | 1 No. | 1 No. |

### Process:

1. Dexlansoprazole, magnesium carbonate, sugar and low substituted hydroxypropyl cellulose were sifted through a suitable screen and mixed well to obtain a dusting powder.
2. Hydroxypropyl cellulose was dissolved in a water to form binder solution.
3. Sugar pellets were layered with the dusting powder obtained in step 1 while spraying the binder solution obtained in step 2.
4. The coating dispersion for the first barrier layer was prepared by dissolving hydroxypropylmethyl cellulose in the water followed by dispersing titanium oxide and talc into the obtained dispersion.
5. The pellets obtained in step 3 were coated with the coating dispersion obtained in step 4.
6. Eudragit® L100, Eudragit® S100 and triethyl citrate were dissolved in the water and ethanol and talc was dispersed into the resulting dispersion to obtain an enteric coating dispersion.
7. The pellets obtained in step 5 were coated with the enteric coating dispersion prepared in step 6 to give enteric coated pellets.
8. The coating dispersion for the second barrier layer was produced by dissolving hydroxypropylmethyl cellulose in the water followed by dispersing titanium oxide and talc into the obtained dispersion.
9. The pellets obtained in step 7 were coated with the coating dispersion obtained in step 8.
10. Dexlansoprazole, magnesium carbonate, sugar and low substituted hydroxypropyl cellulose were sifted and mixed well to obtain a dusting powder for second drug layer.
11. The pellets obtained in step 9 were layered with the dusting powder obtained in step 10 while spraying the hydroxypropyl cellulose solution.
12. The coating dispersion for the third barrier layer was produced by dissolving hydroxypropylmethyl cellulose in the water followed by dispersing titanium oxide and talc into the obtained dispersion.
13. The pellets obtained in step 10 were further coated with the coating dispersionobtained in step 12.
14. To prepare the enteric coating dispersion,triethyl citrate, polysorbate 80 and talc were allowed to disperse in a portion of water and this was added to Eudragit® L30 D55 while stirring and then rest of water was added.
15. The pellets obtained in step 13 were further coated with the enteric coating dispersionobtained in step 14.
16. To the resultant enteric coated pellets, talc and aerosil were added to give lubricated enteric coated pellets which were filled into HPMC capsule (1 No).

### In-Vitro Dissolution Profile:

The formulation of dexlansoprazole DR capsules obtained in Example 5 was subjected to dissolution studies first in 500 ml of 0.1N HCl at 37°C using USP apparatus I (Basket method) at 100 rpm for 2 hours and then in 900 ml of phosphate buffer (pH 7.0) with 5mM SLSat 37°C using USP apparatus I (Basket method) at 100 rpm. Table 2 provides the dissolution profile.

**Table 2:**

| **Time point (min)** | **% Drug dissolved** | |
|---|---|---|
| | **60mg** | **30mg** |
| **Dissolution in 0.1N HCl** | | |
| 120 | <1 | <1 |

| **Dissolution in Phosphate Buffer (pH 7.0)+SLS** | | |
|---|---|---|
| 130 | 19 | 15 |
| 140 | 24 | 20 |
| 160 | 35 | 38 |
| 170 | 52 | 50 |
| 180 | 75 | 70 |
| 195 | 97 | 86 |
| 225 | 100 | 95 |
| 240 | 100 | 96 |

## Claims

1. A pulsatile-release pharmaceutical formulation of dexlansoprazole comprising a single type of enteric coated pellets, wherein said enteric coated pellets comprise:
a) a first enteric portion comprising dexlansoprazole; and
b) a second enteric portion comprising dexlansoprazole, such that said first enteric portion releases dexlansoprazole in the pH range of 6-7.5 and said second enteric portion releases dexlansoprazole in the pH range of 5-6.

2. A pulsatile-release pharmaceutical formulation of dexlansoprazole comprising a single type of enteric coated pellets, wherein said enteric coated pellets comprise:
a) a first enteric portion comprising:
(i) a core comprising dexlansoprazole and one or more pharmaceutically acceptable excipients;
(ii) a barrier layer surrounding the core, said barrier layer comprising a water soluble polymer and one or more pharmaceutically acceptable excipients; and
(iii) an enteric layer surrounding the barrier layer, wherein said enteric layer comprises an enteric polymer and one or more pharmaceutically acceptable excipients; such that said first enteric portion releases dexlansoprazole in the pH range of 6-7.5, and
b) a second enteric portion comprising:
(i) a layer comprising dexlansoprazole and one or more pharmaceutically acceptable excipients;
(ii) a barrier layer surrounding the dexlansoprazole layer, said barrier layer comprising a water soluble polymer and one or more pharmaceutically acceptable excipients; and
(iii) an enteric layer surrounding the barrier layer, wherein said enteric layer comprises an enteric polymer and one or more pharmaceutically acceptable excipients; such that said second enteric portion releases dexlansoprazole in the pH range of 5-6.

3. A pulsatile-release pharmaceutical formulation of dexlansoprazole comprising a single type of enteric coated pellets, wherein said enteric coated pellets comprise:
a) a first enteric portion comprising:
(i) an inert core;
(ii) a drug layer surrounding the inert core comprising dexlansoprazole and one or more pharmaceutically acceptable excipients;
(ii) a barrier layer surrounding the core, said barrier layer comprising a water soluble polymer and one or more pharmaceutically acceptable excipients; and
(iii) an enteric layer surrounding the barrier layer, wherein said enteric layer comprises an enteric polymer and one or more pharmaceutically acceptable excipients; such that said first enteric portion releases dexlansoprazole in the pH range of 6-7.5, and
b) a second enteric portion comprising:
(i) alayer comprising dexlansoprazole and one or more pharmaceuticallyacceptable excipients;
(ii) a barrier layer surrounding the dexlansoprazole layer, said barrier layer comprising a water soluble polymer and one or more pharmaceutically acceptable excipients; and
(iii) an enteric layer surrounding the barrier layer, wherein said enteric layer comprises an enteric polymer and one or more pharmaceutically acceptable excipients; such that said second enteric portion releases dexlansoprazole in the pH range of 5-6.

4. The pulsatile-release pharmaceutical formulation according to claims 1-3, wherein the first enteric portion comprises 40% to 90% of dexlansoprazole and one or more pharmaceutically acceptable excipients.

5. The pulsatile-release pharmaceutical formulation according to claims 1-3, wherein the second enteric portion comprises 10% to 60% of dexlansoprazole and one or more pharmaceutically acceptable excipients.

6. The pulsatile-release pharmaceutical formulation according to claims 1-3, wherein the first enteric portion comprises an enteric polymer at 10% to 50% by weight of barrier layer coated pellets.

7. The pulsatile-release pharmaceutical formulation according to claim 6, wherein the enteric polymer comprises a mixture of methacrylic acid copolymer.

8. The pulsatile-release pharmaceutical formulation according to claim 7, wherein the enteric polymer comprises a mixture of methacrylic acid copolymer Type A and Type B.

9. The pulsatile-release pharmaceutical formulation according to claims 1-3, wherein the second enteric portion comprises an enteric polymer at 1% to 30% by weight of barrier layer coated pellets.

10. The pulsatile-release pharmaceutical formulation according to claim 9, wherein the enteric polymer comprises methacrylic acid copolymer.

11. The pulsatile-release pharmaceutical formulation according to claim 10, wherein the enteric polymer comprises methacrylic acid-ethyl acrylate copolymer.

12. The pulsatile-release pharmaceutical formulation according to claims 1-3, wherein the second enteric portion is either coated either directly over the first enteric portion or it may be separated from the first enteric portion by a barrier layer.

13. The pulsatile-release pharmaceutical formulation according to claims 2-3, wherein the pharmaceutically acceptable excipientscomprise one or more of a binder, a diluent, a surfactant, a lubricant, a disintegrant, a plasticizer, a stabilizer, an opacifier, a solvent, and/or mixtures thereof.

14. The pulsatile-release pharmaceutical formulation according to claim 13, wherein the binder comprises one or more of starches, such as corn starch, pregelatinized starch, maize starch; cellulose derivatives, such as hydroxypropylmethyl cellulose, hydroxypropyl cellulose, hydroxyethyl cellulose, methyl cellulose; gums, such as xanthan gum, gum acacia, gum arabic, tragacanth; water-soluble vinylpyrrolidone polymers, such as polyvinylpyrrolidone, copolymer of vinylpyrrolidone vinyl acetate; sugars, such as sorbitol, mannitol; sodium alginate, propylene glycol, methacrylates, and/or mixtures thereof.

15. The pulsatile-release pharmaceutical formulation according to claim 13, wherein the diluent comprises one or more of sugars, such as dextrose, glucose, sucrose, lactose; sugar alcohols, such as sorbitol, xylitol, mannitol; cellulose derivatives, such as, powdered cellulose, microcrystalline cellulose, silicified microcrystalline cellulose; starches, such as corn starch, pregelatinized starch, maize starch; calcium carbonate, calcium phosphate-dibasic, calcium phosphate-tribasic, calcium sulfate, and/or mixtures thereof.

16. The pulsatile-release pharmaceutical formulation according to claim 13, wherein the surfactant comprises one or more of sodium lauryl sulphate, polysorbates, higher fatty acid ethers, esters, salts, and/or mixtures thereof.

17. The pulsatile-release pharmaceutical formulation according to claim 13, wherein the lubricant comprises one or more of talc, aerosil, magnesium stearate, stearic acid, glyceryl monostearate, glyceryl behenate, sodium stearyl fumarate, sodium starch fumarate, and/or mixtures thereof.

18. The pulsatile-release pharmaceutical formulation according to claim 13, wherein the disintegrant comprises one or more of alginic acid or alginates, microcrystalline cellulose, hydroxypropyl cellulose, carmellose, croscarmellose sodium, crospovidone, sodium starch glycolate, starch, pregelatinized starch, carboxymethyl starch, polyacrylates, and/or mixtures thereof.

19. The pulsatile-release pharmaceutical formulation according to claim 13, wherein the plasticizer comprises one or more of triethyl citrate, polyethylene glycol, triacetin, tributylsebecate, diethyl phthalate, dimethyl phthalate, propylene glycol, and/or mixtures thereof.

20. The pulsatile-release pharmaceutical formulation according to claim 13, wherein the stabilizer comprises one or more of basic inorganic salt of magnesium including heavy magnesium carbonate, magnesium carbonate, magnesium oxide, magnesium hydroxide, magnesium metasilicate aluminate, magnesium silicate aluminate, magnesium silicate, magnesium aluminate, synthetic hydrotalcite and aluminum magnesium hydroxide; basic inorganic salts of calcium including precipitated calcium carbonate and calcium hydroxide; basic inorganic salts of sodium including sodium carbonate and sodium hydrogen carbonate; potassium basic inorganic salts, such as potassium carbonate; aluminum basic inorganic salts, such as aluminum silicate, and/or mixtures thereof.

21. The pulsatile-release pharmaceutical formulation according to claim 13, wherein the opacifier comprises one or more of titanium dioxide, iron oxide, zinc oxide, and/or mixtures thereof.

22. The pulsatile-release pharmaceutical formulation according to claim 13, wherein the solvent comprises one or more of water, methanol, ethanol, acidified ethanol, acetone, diacetone, polyols, polyethers, oils, esters, alkyl ketones, methylene chloride, isopropyl alcohol, butyl alcohol, methyl acetate, ethyl acetate, isopropyl acetate, castor oil, ethylene glycol monoethyl ether, diethylene glycol monobutyl ether, diethylene glycol monoethyl ether, dimethylsulphoxide, dimethylformamide, tetrahydrofuran, and/or mixtures thereof.

23. The pulsatile-release pharmaceutical formulation according to claims 1-3 may be used with one or more additional active ingredients selected from the group consisting of an anti*-Helicobacter pylori* agent, an imidazole compound, a bismuth salt, a quinoline compound, and combinations thereof.

24. The pulsatile-release pharmaceutical formulation according to claims 1-3 further comprising one or more additional active ingredients selected from the group consisting of anti*-Helicobacter pylori* agents, an imidazole compound, a bismuth salt, a quinoline compound, and combinations thereof.

25. A process to prepare a pulsatile-release pharmaceutical formulationof dexlansoprazole, wherein the process involves the steps of:
1) preparing the core by mixing dexlansoprazolewith one or more pharmaceutically acceptable excipients;
2) coating the core obtained in step 1 with a barrier layer comprising a water soluble polymer and one or more pharmaceutically acceptable excipients;
3) coating the resultant core of step 2 with an enteric layer to form enteric coated pellets;
4) coating the enteric coated pellets with another enteric layer by introducing an intermediate barrier layer and/or dexlansoprazole layer in between the two enteric layers; and
5) filling of the pellets obtained in step 4 into capsule after lubrication.

26. The process according to claim 25, wherein the core is prepared either by granulation, direct blending or extrusion/spheronization.

27. A process to prepare a pulsatile-release pharmaceutical formulationof dexlansoprazole, wherein the process involves the steps of:
1) mixing dexlansoprazole and one or more pharmaceutically acceptable excipients to obtain a dusting powder;
2) coating an inert core with the dusting powder obtained in step 1, while spraying a binder solution to obtain dexlansoprazole core;
3) coating the core obtained in step 2 with a barrier layer comprising a water soluble polymer and one or more pharmaceutically acceptable excipients;
4) coating the resultant core of step 3 with an enteric layer to form enteric coated pellets;
5) coating the enteric coated pellets with another enteric layer by introducing an intermediate barrier layer and/or dexlansoprazole layer in between the two enteric layers;and
6) filling of the pellets obtained in step 5 into capsule after lubrication.
